# EUROPEAN PATENT APPLICATION

(11) **EP 0 569 081 A1**
(43) Date of publication of application: **10.11.1993**
(21) Application number: 93201217.2
(22) Date of filing: 28.04.1993
(51) Int. Cl.: G01N 33/58, G01N 33/74, C12Q 1/28

(54) **Signal generating reagent**

(30) Priority: 02.05.1992 GB 9209571
(71) Applicant: Johnson & Johnson Clinical Diagnostics, Inc., Rochester New York 14650 (US)
(72) Inventor: Lingham, Ian Nigel, c/o KODAK LIMITED, Harrow, Middlesex HA1 4TY (GB); Moore, Patricia, c/o KODAK LIMITED, Harrow, Middlesex HA1 4TY (GB); Summers, Malcolm Robert, c/o KODAK LIMITED, Harrow, Middlesex HA1 4TY (GB)
(74) Representative: Mercer, Christopher Paul

(57) **Abstract**

A signal generating reagent (SGR) for use in an immunoassay comprising a luminescence substrate and an enhancer, characterized in that it comprises an inorganic azide in an amount in the range 0.001% ^{w}/v to 0.01% ^{w}/v. A method for the immunoassay of a biological specimen using the SGR, use of the SGR and a kit comprising inter alia the SGR are also claimed.

## Description

This invention relates to an improved reagent ( hereinafter referred to as a signal generating reagent-SGR- ) for measuring the activity of peroxidase labels used in immunoassays of biological specimens, such as body fluids, to a method for assaying biological specimens using the reagent, to the use of the reagent in an assay and to a kit for assaying biological specimens which comprises the reagent.

In recent years a number of hospital laboratories have observed anomalous values obtained when analysing body fluids, such as blood samples, for moieties such as hormones by means of immunoassays utilising peroxidase labels. These anomalous values have occasionally been observed, for example in assays for thyroid stimulating hormone (TSH) using immunoassays such as the enhanced luminescence immunoassays of European Patent No 87959. Blood samples taken from hyperthyroid patients which should have undetectable TSH sometimes give positive results in the immunoassay when measurements of light emission are taken a short period such as 2 minutes, after addition of the peroxidase detection reagent. The problem can be sufficiently serious for a toxic patient to be wrongly classified as euthyroid. When light emission measurements are made after a longer interval from the addition of the peroxidase detection reagent the size of the incorrect signal gradually decays until it is undetectable, usually after about 20 minutes. Various measures can be taken to reduce or remove the problem such as taking measurements after a longer interval of time, e.g. 20 minutes; freezing and thawing or centrifuging the biological samples before use in the immunoassay. However these solutions are inconvenient and unacceptable as a long term measure. Although the problem has been observed particularly in analyses for TSH, it has been encountered in analyses for other moieties such as alpha-fetoprotein (AFP) and antibodies to hepatitis B surface antigen (HBs). With these other moietes however the problem is only occasionally sufficiently serious to cause clinical misclassification.

We have now found that the problem can be significantly reduced or eliminated by use of a modified peroxidase detection, or signal generating reagent (SGR). This modified signal generating reagent can be used to detect peroxidase labels in immunoassays such as the enhanced luminescent or luminometric assays described in European Patent Specifications Nos. EP 87959 and EP 116454 although the modification can also be applied to other reagents for detecting peroxidase activity, eg reagents in which a coloured or fluorescent product is developed.

According to the present invention we provide a signal generating reagent (SGR) for use in an immunoassay comprising a luminescence substrate and an enhancer, characterized in that it comprises an inorganic azide in an amount in the range 0.001% ^{w}/v to 0.01% ^{w}/v.

Further according to the invention we provide a method for the immunoassay of a biological specimen which comprises a step in which the specimen is contacted with a binding reagent and thereafter a measurement is made using a signal generating reagent (SGR), characterized in that the signal generating agent comprises an inorganic azide in an amount in the range 0.001% ^{w}/v to 0.01% ^{w}/v.

Further according to the invention we provide the use, in an immunoassay of a biological specimen, of one or more binding reagents ( optionly together with other materials ) and a signal generating reagent (SGR), characterized in that the signal generating reagent comprises an inorganic azide in an amount in the range 0.001% ^{w}/v to 0.01% ^{w}/v.

Further according to the present invention we provide a kit useful in an immunoassay and comprising a label and a signal generating reagent, characterized in that the signal generating reagent comprises an inorganic azide in an amount in the range 0.001% ^{w}/v to 0.01% ^{w}/v.

There are various ways in which the components and active compounds can be included in the signal generating reagent of the invention. These will depend on the details of the method to be used or of the assay to be performed and will be known to workers in this field. However a preferred form of the SGR for many purposes comprises a liquid buffer solution containing the azide together with two solid further components, suitably both in tablet form, i.e. as tablets A and B, each of which tablets contains at least one active compound. In a preferred form the first active ingredient contained in tablet A contains two active ingredients suitably sodium luminol and para iodophenol whilst tablet B is a perborate ( such as sodium perborate ). In use tablets A and B are dissolved in the buffer solution.

Any soluble inorganic azide may be included in the buffer solution. Suitable azides include alkali metal azides such as sodium azide NaN₃. The range of inclusion of the azide is in the range 0.001% ^{w}/v to 0.01% ^{w}/v, preferably in the range 0.001% to 0.007% and particularly around 0.005%. Inclusion of aside in an amount significantly exceeding 0.01% can have a deleterious effect on the enzyme, e.g. horseradish peroxidase, used in the assay. Suitable azides will be those which do not interfere in the assay.

The assay method of the invention may be used to assay biological specimens, such as blood and other physiological fluids for a wide variety of materials such as, antibodies, hormones, drugs and proteins.

The invention can be used in conjunction with a large number of enhanced luminescent and luminometric assays, particularly chemiluminescent assays and including those described in European Patent Specifications Nos. Ep 87959 and EP 116454. It is particularly useful in connection with assays for thyroid stimulating hormone (TSH). One such assay is the Amerlite ^{(TM)} TSH 60 assay. This uses an immunometric assay technique based on enhanced luminescence for measurement of TSH in human serum or plasma. In the assay TSH in the sample reacts simultaneously with an enzyme-labelled antibody and an antibody coated onto the walls of a well. Unbound material is then removed by aspiration and washing. The activity of the enzyme bound to the surface of the wells conjugate is measured by an enhanced luminescence reaction. A SGR containing a luminescence substrate and an enhancer is added to the coated wells to initiate a light-emitting reaction. The purpose of the enhancer is to increase the level of light produced and to prolong its emission. Readings are taken of the light signals.

In the Amerlite ^{(TM)} assay described above the enzyme label is horseradish peroxidase (HRP). Similarly any suitable luminescence substrates may be used although, in the assay described above, luminol derivatives and peracid salts are preferred. Suitable enhancers include substituted phenols such as paraiodophenol.

In the assay described above it is believed that peroxidase enzymes related to HRP (the enzyme label frequently used) which occur in blood cells contaminate the sample on and are adsorbed onto the surface of the reaction vessel during the immunoassay procedure and give a spurious signal thereby causing the problem which the present invention seeks to overcome.

Azides are normally poisons or inhibitors of peroxidases and have been used in histochemistry to remove non-specific peroxidase-like activity. We have unexpectedly found that in immunoassay low levels of azide can inhibit non-specific peroxidase activity whilst not interfering with the activity of the specific peroxidase immunoassay label which is present at the same time. The aside is then removed by washing before the specific peroxidase label is used. The azide does not therefore come into contact with the specific peroxidase label.

The invention is illustrated by the following examples:
The advantages accruing from the inclusion of sodium aside in SGR buffer are illustrated by Examples 1-3 below which compare tests carried out with and without azide addition to the buffer. The tests were carried out in a manner outlined above for the preferred form of the invention.

### Example 1

Measurement of alpha-fetoprotein using the Amerlite ™ AFP-2T assay.

This test was carried out in accordance with the protocol described in pack leaflet LAN 0152/2152 which accompanies the test kit supplied by Kodak Clinical Diagnostics Limited (KCDL).

AFP is a specific fetal alpha-globulin which passes into the amniotic fluid during pregnancy. Elevated ranges of AFP indicate the existance of a number of fetal abnormalities including for example spina bifida.

In the example five different test samples were assayed using signal generating reagents (SGR) both with and without azide in the SGR buffer. Measurements were taken in both cases 2 and 20 minutes after addition of the SGR. The results are set out in the Table.

The steps carried out in the assay were as follows:
1. The equipment to be used, i.e. the shaker incubator, workstation, washer and analyser (all Amerlite ™) were assembled ready to be used.
2. A strip holder was assembled with sufficient wells to enable the running of all standards, controls and specimens as required.
3. 150µl of blue conjugate reagent was dispensed into each coated well without contaminating the well rims.
4. 50µl of standards, controls and specimens was pipetted into appropriate wells. This led to a colour change from blue to blue/green.
5. The wells were covered with a lid and shaken and incubated at 37°C for 1 hour in the shaker incubator.
6. Using the washer, all wells were aspirated and washed. Care was taken to avoid handling the tops of the wells both before and after filling.
7. 250µl of SGR was dispensed immediately into all coated wells.
8. Readings of wells were taken using the Amerlite™ analyser 2 and 20 minutes after SGR addition.

### Example 2

Measurement of thyroid stimulating hormone using the Amerlite ™ TSH -60 assay.

This test was carried out in accordance with the protocol described in pack leaflet LAN 0008/2008 which accompanies the test kit supplied by KCDL.

Thyroid stimulating hormone (TSH) occurs in human serum and is measured to aid in the differential diagnosis of thyroid disease.

The procedure of Example 1 was followed except in respect of steps 3 and 4 of the assay.

Steps 3 and 4 in the assay of this example were as follows:
3. 100µl of standards, controls and specimens was pipetted into appropriate wells.
4. 100µl of the conjugate reagent was dispensed into each coated well without contaminating the well rims. This led to a colour change from yellow to blue/green.

The results are set out in the Table.

### Example 3

Measurement of an antibody using the Amerlite ™ anti-HBs (antibody to Hepatitis B surface antigen) assay. This test was carried out in accordance with the protocol described in pack leaflet LAN 1203 which accompanies the test kit supplied by KCDL.

This test is carried out to measure the antibody to hepatitis B surface antigen in human serum and plasma. The measurement can serve as a marker for hepatitis B virus (HBV) infection - a cause of liver disease.

The steps in the assay which vary somewhat from those of Example 1 are set out below.
1. As Example 1
2. As Example 1
3. 200µl of standards and specimens was pipetted into appropriate wells.
4. The wells were covered with a lid and shaken and incubated at 37°C for 2 hours in the shaker incubator.
5. All wells were aspirated and washed using Amerlite™ Serology Wash Reagent in the washer, care being taken to avoid handling the tops of the wells both before and after filling.
6. 200µl of conjugate reagent was immediately dispensed into all coated wells without contaminating the well rims.
7. The wells were again covered by a lid and shaken and incubated at 37°C for 2 hours in the shaker incubator.
8. All wells were aspirated and washed as in step 5.
9. Readings of wells were taken as in step 8 of Example 1.

The results set out in the Table show that with no azide present readings of light emission after 20 minutes are substantially less than those after 2 minutes. However with azide present readings after only 2 minutes are lower than those for no azide after even 20 minutes. This demonstrates the effectiveness of operation in accordance with the invention.

Please note that the test kits used in the above and now supplied by KCDL were formerly supplied by Amersham International.

**TABLE**

| **Analyte** | **Units** | **Expected Value** | **Measured Value** | | | |
|---|---|---|---|---|---|---|
| | | | **No azide** | | **Plus azide** | |
| | | | **2min** | **20min** | **2min** | **20min** |
| AFP Example 1 | mU/l | <9.0 | 35.9 | 11.5 | 5.7 | 6.2 |
| | | <9.0 | 27.4 | 12.0 | 5.4 | 5.3 |
| | | <9.0 | 11.7 | 3.6 | 2.5 | 3.2 |
| | | <9.0 | 8.5 | 5.2 | 3.2 | 3.9 |
| | | <9.0 | 8.4 | 3.1 | 2.2 | 2.6 |
| TSH Example 2 | mIU/l | 0.2 to 2.9 | 3.15 | 2.25 | 2.01 | 2.07 |
| | | 0.2 to 2.9 | 2.35 | 1.73 | 1.52 | 1.54 |
| | | <0.1 | 0.17 | 0.07 | 0.05 | <0.04 |
| | | <0.1 | 0.14 | 0.04 | <0.04 | <0.04 |
| | | <0.1 | 0.13 | 0.04 | <0.04 | <0.04 |
| aHBs Example 3 | mIU/ml | <10 | 28.0 | 3.7 | 0 0 | |
| | | <10 | 13.0 | 1.5 | 0 0 | |
| | | <10 | 12.1 | 0 | 0 0 | |
| | | <10 | 7.2 | 0.1 | 0 0 | |
| | | <10 | 6.8 | 0 | 0 0 | |

## Claims

1. A signal generating reagent (SGR) for use in an immunoassay comprising a luminescence substrate and an enhancer, characterized in that it comprises an inorganic azide in an amount in the range 0.001% ^{w}/v to 0.01% ^{w}/v.

2. A signal generating reagent according to claim 1 characterized in that the enhancer is a substituted phenol.

3. A signal generating agent according to claim 1 or claim 2 characterized in that the azide is sodium azide.

4. A signal generating agent according to any one of the preceding claims characterized in that the azide is present in an amount in the range 0.001% to 0.007% ^{w}/v.

5. A method for the immunoassay of a biological specimen which comprises a step in which the specimen is contacted with a binding reagent and thereafter a measurement is made using a signal generating reagent (SGR), characterized in that the signal generating agent comprises an inorganic azide in an amount in the range 0.001% ^{w}/v to 0.01% ^{w}/v.

6. A method according to claim 5 characterized in that it is an assay for thyroid stimulating hormone (TSH).

7. The use, in an immunoassay of a biological specimen, of one or more binding reagents and a signal generating reagent (SGR), characterized in that the signal generating reagent comprises an inorganic azide in an amount in the range 0.001% ^{w}/v to 0.01% ^{w}/v.

8. A kit useful in an immunoassay and comprising an enzyme label and a signal generating reagent, characterized in that the signal generating reagent comprises an inorganic azide in an amount in the range 0.001% ^{w}/v to 0.01% ^{w}/v.

9. A kit according to claim 8 characterized in that the enzyme label is a peroxidase.

10. A kit according to claim 9 characterized in that the peroxidase is horseradish peroxidase (HRP).
